# EUROPÄISCHE PATENTSCHRIFT

(11) **EP 0 613 497 B1**
(45) Veröffentlichungstag und Bekanntmachung des Hinweises auf die Patenterteilung: **16.07.1997**
(21) Anmeldenummer: 92923662.8
(22) Anmeldetag: 16.11.1992
(51) Int. Cl.: C12N 15/12, C07K 14/00, C12N 15/62, C12N 5/10, A01K 67/027, C12N 15/73, C12P 21/08

(54) **LYMPHOIDES CD30-ANTIGEN**
LYMPHOID CD30-ANTIGEN
ANTIGENE LYMPHOIDE CD30

(30) Priorität: 15.11.1991 DE 4137716; 02.01.1992 DE 4200043
(43) Veröffentlichungstag der Anmeldung: 07.09.1994
(73) Patentinhaber: Medac Gesellschaft für klinische Spezialpräparate mbH, D-20354 Hamburg (DE)
(72) Erfinder: STEIN, Harald, D-1000 Berlin 33 (DE); DÜRKOP, Horst, D-14129 Berlin (DE); LATZA, Ute, D-1000 Berlin 19 (DE)
(74) Vertreter: Benedum, Ulrich Max, Dr.
(86) Internationale Anmeldenummer: DE9200956
(87) Internationale Veröffentlichungsnummer: WO9310232

(56) Entgegenhaltungen:
- JOURNAL OF IMMUNOLOGY, Bd. 239, Nr. 6, 15. September 1987, BALTIMORE, US, Seiten 2081-2087; Froese, P. et al.: 'Biochemical characterization and biosynthesis of the Ki-1 antigen in Hodgkin-derived and virus-transformed human B and T lymphoid cell lines'
- CELL, Bd. 68, Nr. 3, 7. Februar 1992, CAMBRIDGE, NA, US, Seiten 421-427; Durkop H, Latza U, Hummel M, Eitelbach F, Seed B, Stein H.: 'Molecular cloning and expression of a new member of the nerve growth factor receptor family that is characteristic for Hodgkin's disease.'

## Beschreibung

Die Erfindung betrifft das bei Morbus Hodgkin auftretende, lymphoide Oberflächenantigen CD30, dessen Protein- und die zugehörige Nucleotidsequenz, seine gentechnische Herstellung, Mittel zur Diagnose und Untersuchung des Morbus Hodgkin sowie die Verwendung der Nucleotidsequenzen, insbesondere zur Schaffung transgener Tiere.

Die Pathogenese des Morbus Hodgkin (HD), das in Mitteleuropa zu den häufigen humanen Lymphomen zählt, ist noch weitgehend unverstanden. Mit der Charakterisierung des Oberflächenantigens CD30 (Froese et al. (1987) J. Immunol, 139, 2081-2087) wurde die Identifikation der Tumorzellen des Morbus Hodgkin, die Hodgkinschen (H) und die Reed-Sternberg-Zellen (RS), wesentlich erleichtert oder überhaupt erst möglich (Schwab et al. (1982) Nature 299, 65-67; Stein et al. (1982) Int.J.Cancer 30, 445-449; McMichael, Hrsg.(1987): *Leukocyte Typing III,* Oxford University Press, Oxford). Daß das Auftreten des CD30-Oberflächenantigens mit der Aktivierung der Lymphozyten einhergeht, haben Untersuchungen an *in vitro* stimulierten oder viral - durch HTLV-I;HTLV-II, EBV - transformierten T und B-Zellen gezeigt (Stein et al, (1985) Blood, 66, 848-858; Andreesen et al. (1984) Blood, 63, 1299-1302). Es wird vermutet, daß Hodgkin- und Reed-Sternberg-Zellen maligne transformierte T oder B-Zellen sind und daß die histologischen Varianten beim Morbus Hodgkin auf der Sekretion unterschiedlicher Cytokine beruhen (Stein et al. (1989) Recent Results Cancer Res., 117, 15-26.). Das CD30-Antigen scheint somit für das interzelluläre Signalsystem und die Tumorgenese wichtig zu sein.

Die histologischen Untersuchung an den Tumorgeweben ergaben ferner, daß das CD30-Antigen auch zur Identifizierung von großzelligen anaplastischen Neoplasmen geeignet ist, welche deshalb auch als CD30- oder Ki-1-positive anaplastische großzellige Lymphome (ALCL: engl. Ki-1⁺ anaplastic large cell lymphoma) bezeichnet werden. Diese besonders bei Kindern und Jugendlichen auftretende Tumore bilden eine separate Spezies von Lymphomen und werden wahres histiocytisches Lymphom (engl.: true histiocytic lymphoma) oder bösartige Histiocytose (engl.: malignant histiocytosis) genannt. Das gleichzeitige Auftreten von re-arrangierten Ig- und T-Zell-Rezeptorgenen in der Mehrzahl der CD30-positiven ALCLs zeigt, daß diese Tumorzellen lymphoid sind. Die CD30-positiven ALCLs bilden deshalb nicht nur phäno-, sondern auch genotypisch eine Einheit. CD30-positive ALCLs werden in primär und sekundär unterteilt; sie stammen zumeist von T-Zellen und Null-Zellen - weniger von B-Zellen - ab. Morphologisch werden die ALCLs wie folgt typisiert: a) common; b) Hodgkin-related; c) giant cell-rich und d) lymphohistiocytisch.

Zur Detektierung des CD30-Antigens standen bislang - definitionsgemäß - nur monoklonale Antikörper, insbesondere die monoklonalen Antikörper Ki-1 und Ber-H2, zur Verfügung (Schwab et al. (1982) Nature 299, 65-67; Schwarting et al (1989) Blood 74, 1678-1689). Hierbei ist jedoch nachteilig, daß sie nur jeweils ein Epitop auf dem Antigen erkennen; auch sind sie nicht zur Detektierung und Untersuchung der zugehörigen Nucleinsäuren geeignet. Eine Diagnostik, die nur auf einzelnen Epitopen basiert, ist auch gegenüber Artefakten anfällig und nicht umfassend. Schließlich hängt die Erkennbarkeit eines Epitops von der Fixierung und Präparierung der Probe ab; auch können sich diese Epitope mit dem Status der Zelle ändern. So kann ein Epitop durch Glykoslierung von benachbarten N- oder O-Glykosylierungsstellen blockiert werden, und auch Phosphorylierungen, insbesondere Tyrosin-Phosphorylierungen, etc., bewirken Konformationsänderungen, die ein Epitop, insbesondere wenn es sich aus mehreren Peptidkettenabschnitten zusammensetzt, verschwinden lassen. Solche Änderungen im Zellstatus treten besonders häufig mit dem Fortschreiten des Tumors auf.

Ferner konnte das CD30-Antigen bislang nicht näher auf seine Eigenschaften als Rezeptor- bzw. Bindungsprotein hinsichtlich anderer Wachstums- und hormoneller Faktoren untersucht werden - einerseits wegen der geringen zur Verfügung stehenden Mengen, andererseits wegen seiner Eigenschaft als Membranprotein mit funktionell verschiedenen cytosolischen, transmembranen und extrazellulären Proteinabschnitten, die nachstehend auch Domänen genannt werden.

Der Erfindung liegt deshalb das Problem zugrunde, nicht auf monoklonalen Antikörpern basierende Mittel zur Untersuchung, Diagnose und Behandlung des Morbus Hodgkin und der anaplastischen großzelligen Lymphomen zur Verfügung zu stellen. Insbesondere sollen Möglichkeiten zur Untersuchung der Proteinfunktionen, der Tumorentstehung und Mittel zur Behandlung dieser Krankheiten geschaffen werden.

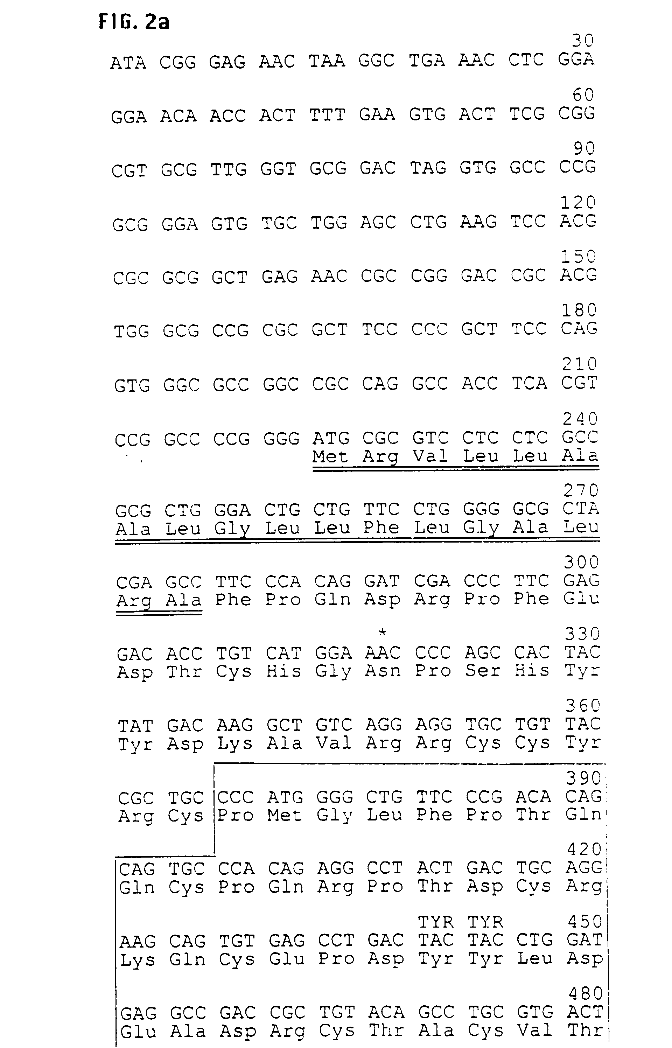

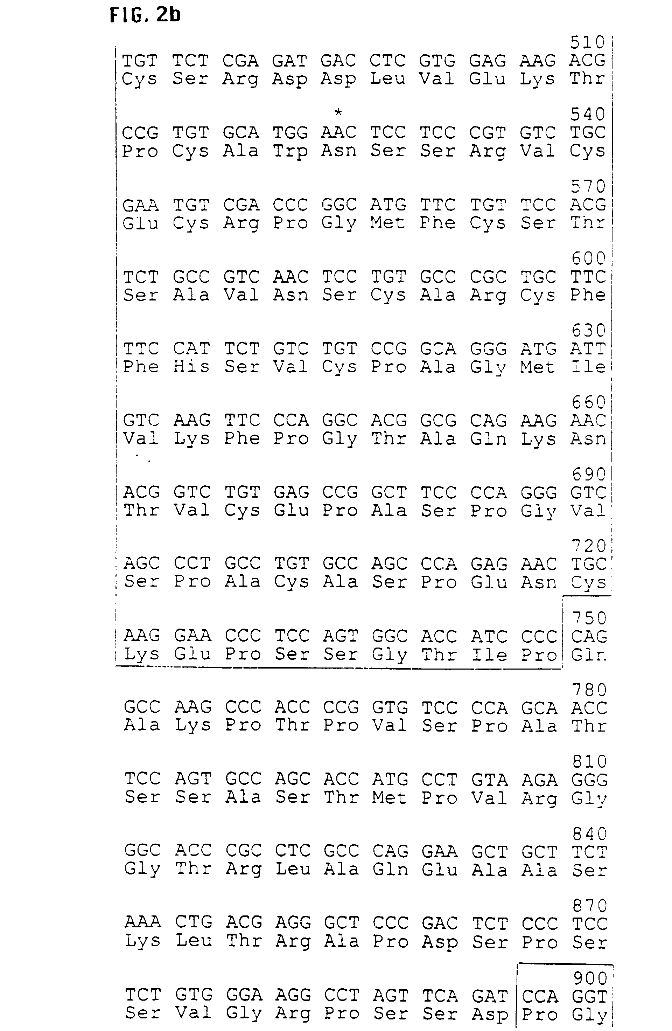

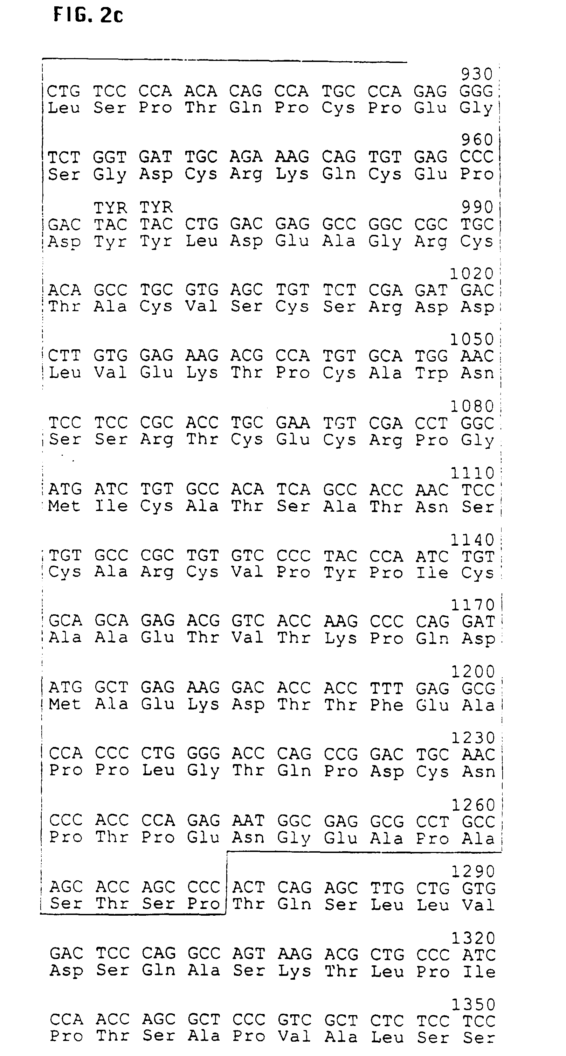

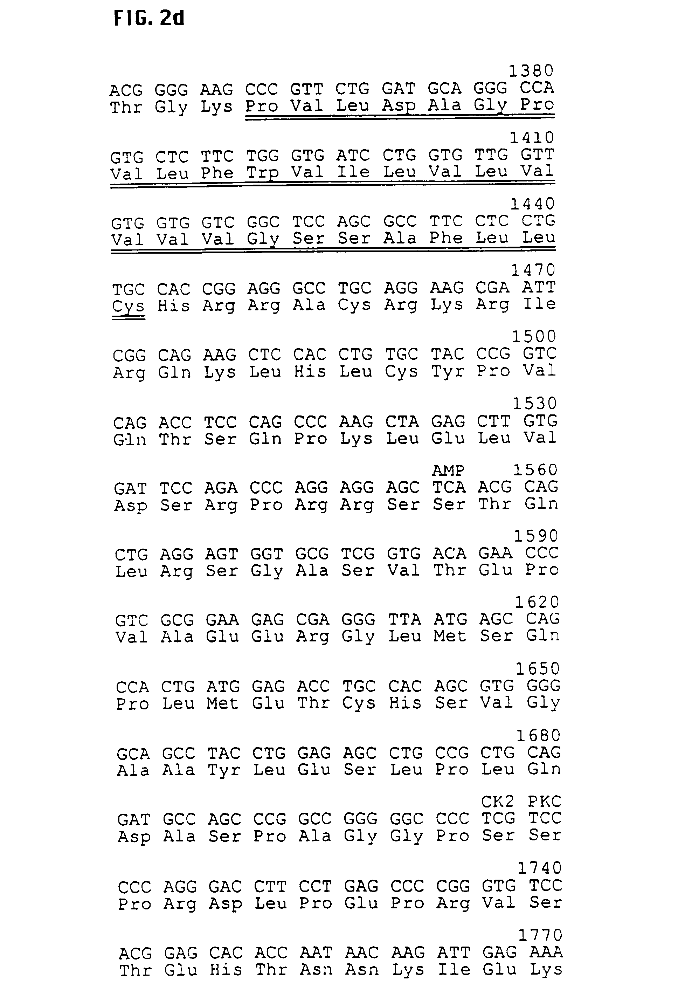

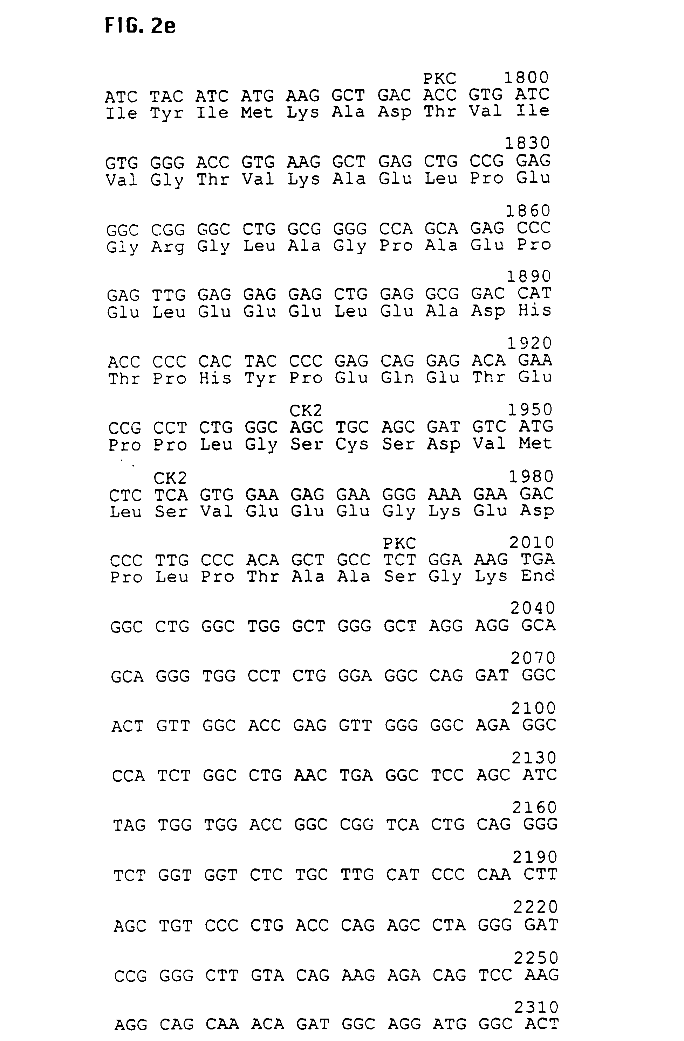

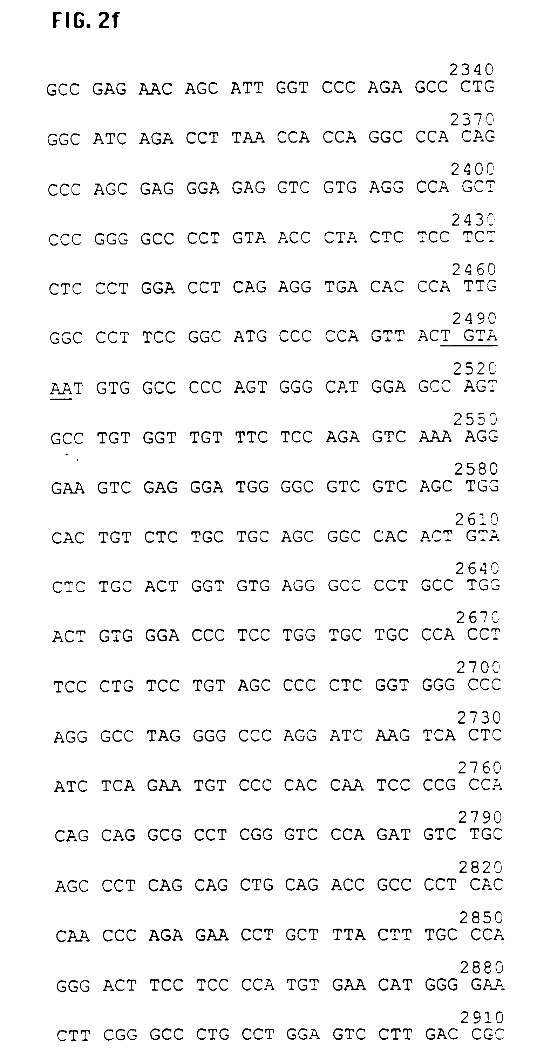

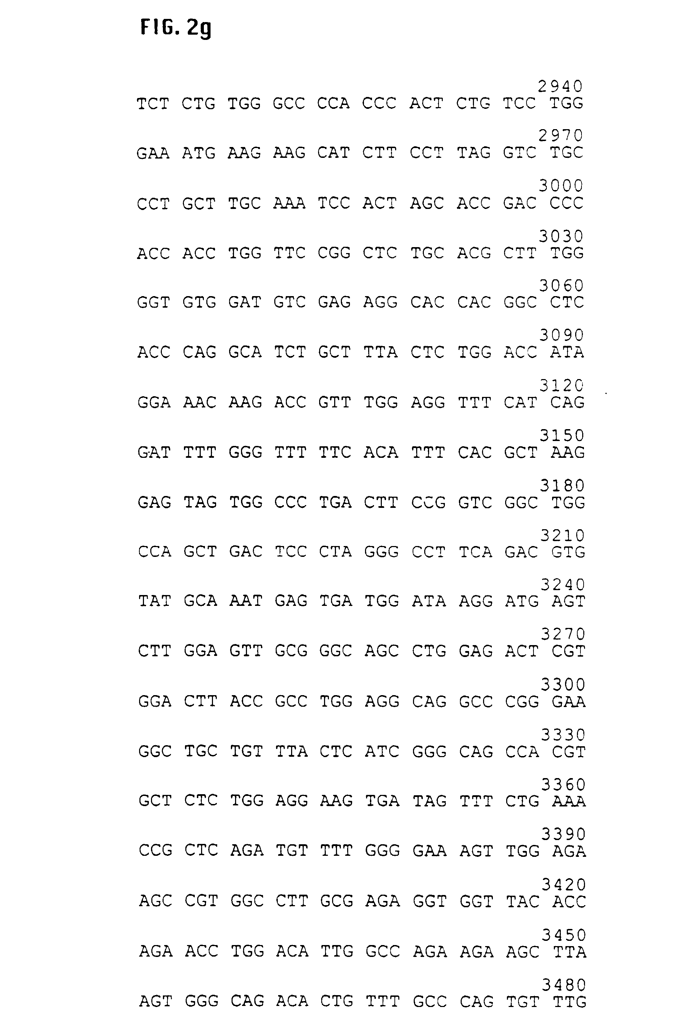

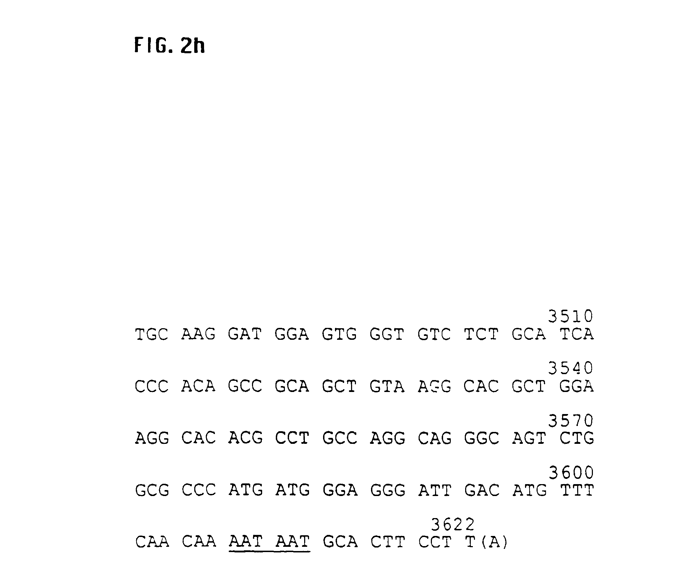

Dieses Problem wird gelöst durch die Bereitstellung einer Nucleinsäure nach Anspruch 1 oder 2, und insbesondere durch eine Nucleotidsequenz, die im wesentlichen der Figur 2 gezeigten Sequenz der CD30-cDNA entspricht bzw. durch eine Nucleotidsequenz, die ein Protein kodiert, dessen Aminosäuresequenz im wesentlichen der in Figur 2 gezeigten Aminosäuresequenz des CD30-Antigen entspricht. Vorteilhafte Ausführungsformen und Weiterbildungen der Erfindungen sind der Beschreibung, den Beispielen, den Unteransprüchen 3 bis 27 und den anliegenden Abbildungen zu entnehmen.

Die erfindungsgemäße Nucleinsäure kann laboranalytisch feststellbare Modifizierungen aufweisen, wie radioaktive Atome, fluoreszierende Farbstoffgruppen (Hawkins & Sulston (1990) *Technique,* Dec., 307) und/oder eingeführte Epitope für auf Licht basierende Systeme zum Nachweis von Nucleinsäuren. Die erfindungsgemäßen Nucleinsäuren können In einer bevorzugten Ausführungsform an einem makroskopischen Träger - wie Beads, insbesondere Glas- und Sepharosekörper, Membranfilter, Nitrocellulosefilter, Nylonfilter, etc. - gebunden sein, um unter anderem dadurch CD30-RNA leichter durch Hybridisierung isolieren bzw anreichern zu können.

Ein Aspekt der Erfindung sind ferner Klonierungsvektoren, die eine derartige DNA-Sequenz enthalten. Besonders geeignete Klonierungsvektoren sind unter anderem solche, mit denen die erfindungsgemäßen Nucleinsäuren amplifiziert werden können, z.B. pBR322, pUR101, M13mp10, M13mp11, M13mp18, M13mp19, pGEM1, etc.; ferner Vektoren, die eine Protein-Exprimierung der klonierten DNA-Sequenzen in den Wirtszellen herbeiführen - z.B Lambda gt11, pATH, etc., ferner Vektoren, die prokaryotische oder eukaryotische Wirtszellen transiert transformieren, bspw. pCDM8 und Derivate, pRC/CMV, etc; ferner Vektoren, die eukaryotische Wirtszellen stabil transformieren und in das Wirtsgenom integrieren, bspw. pXT1, etc; ferner Vektoren, die zu integration in das Genom von Keimbahnzellen geeignet und somit zur Herstellung transgener Tieren geeignet sind, z.B. pSV2Neo, pRSVNeo, pMC1Neo, etc.

Die erfindungsgemäße Lehre umfaßt auch Wirtszellen, die CD30-Antigen, ein Fragment des CD30-Antigens, ein Hybridprotein mit CD30-Antigen oder Mutationen davon exprimieren. Insbesondere eukaryotische Wirtszellen, die CD30-Antigen in die äußere Zellmembran integrieren, mit posttranslationalen Modifikationen versehen, z.B. das CD30-Antigen in natürlicher Weise Glykosylieren bzw. mit komplexen Zuckermolekülen versehen, z.B die in Beispiel 3 beschriebenen COS-Zellen, oder L929 Zellen, RK13 Zellen, WOP Zellen, R9ab Zellen.

Derartige durch rekombinante DNA-Verfahren hergestellte CD30-Moleküle sind in einer Ausführungsform der Erfindung bevorzugte Immunogene zur Herstellung von poly- und monoklonalen Antikörper gegen das lymphoide CD30-Oberflächenantigen. So können z.B. Hybridprotein mit β-Galaktosidase und CD30-Antigenfragmenten aus den nachstehend beschriebenen Lambda-gt-11-Klonen isoliert und als Immunogen verwendet werden. Darüber hinaus ist es durch die beschriebenen Verfahren möglich, das CD30-Antigen in eukaryotischen Zellen zu exprimieren und nach seiner Aufreinigung bzw. als membranständiges Protein mit samt der überexprimierenden Zelle zu Immunisierung zu verwenden. Ferner können chemisch CD30-Peptide gemäß der in Figur 2 gezeigten Proteinsequenz synthetisiert, ggf. mit Glutaraldeyd oder einem anderen Kopplungsreagenz an einem geeigneten Träger - z.B. Thyreoglobulin, Albumin - gekoppelt, und als Immunogen zusammen mit Freundschem Adjuvanz in ein geeignetes Tier - z.B. Maus, Kaninchen, Ziege, Schaf, etc. - injiziert werden. Die bio- und chemosynthetisch hergestellten CD30-(Hybrid)-Moleküle sind auch zur Verwendung in sogenannten Sandwich-lmmunoabsorptionstest geeignet.

Ein weiterer Aspekt der Erfindung besteht darin, daß mit der Kenntnis der Nucleotid- und Proteinsequenz auch die pharmakologisch relevanten Teile von CD30 offenkundig sind. Pharmakologische Bedeutung erlangen insbesondere die extrazellulären Bereiche des CD30-Moleküls, da zu therapeutischen Zwecken verabreichte Ligandenmoleküle zu CD30 naturgemäß zunächst am extrazellulären Teil des Membranproteins binden. Die extrazellulären Bereiche des CD30 werden, wie Vergleichsstudien, die Hydropathiekurve und in-vitro-Transkriptions/Translationsuntersuchungen zeigen, im wesentlichen von der cDNA-Sequenz zwischen Position 277 und Position 1359 kodiert, also der Sequenz zwischen ²⁷⁷-TTCCCACAGGATCGACCC ... // ... CTC CCACGGGGAAG-₁₃₅₉. Es liegt somit nahe, insbesondere diese Bereiche des CD30-Moleküls oder Unterabschnitte davon für die Entwicklung von therapeutischen Mitteln zu verwenden. So sind insbesondere Fusionsproteine mit ursprünglich extrazellulären CD30-Abschnitten besonders geeignet, um natürliche und synthetische Immunotoxine gegen die Hodgkin- und Reed-Sternberg-Zellen zu isolieren oder anzureichern. Ferner können nach extrazellulären Aminosäuresequenzen des CD30 hergestellte Oligopeptide oder rekombinante Peptide als Immunogene zur Herstellung von polyklonalen Antikörpern gegen extrazelluläres CD30 verwendet werden. Auch können synthetisch Liganden zu diesen Proteinsequenzen erzeugt oder entwickelt werden. Diese Liganden oder Antikörper können mit einem Toxin, wie Saporin oder Ricin-A, gekoppelt werden, so daß therapeutisch einsetzbare Immunotoxine zur Behandlung dieser Lymphome erhalten werden können. Ein Aspekt der Anmeldung ist somit die Verwendung der extrazellulären CD30-Sequenzen, zur Herstellung, Suche oder Isolierung von natürlichen - *in vivo* bereits gegebenen - oder artifiziellen - *in vitro* hergestellten oder *in vivo* induzierten - Bindungspartnern, insbesondere von Liganden zur Herstellung von Immunotoxinen. Besondere Bedeutung erlangen hierbei polyklonale Antikörper gegen extrazelluläre CD30-Sequenzen, so daß nunmehr auch erstmals polyklonale Immunotoxine gegen Morbus Hodgkin und anaplastische großzellige Lymphome zur Verfügung stehen. Die extrazellulären CD30-Peptidsequenzen können durch chemische Synthese, insbesondere Festphasen-Peptidsynthese, oder als rekombinantes Fusionsprotein in prokaryotischen und eukaryotischen Zellen hergestellt werden. Bevorzugt ist die Expression in Hefen und eukaryotischen Zellen, insbesondere in Insektenzellen, oder in Gewebekultur mit Fibroblasten, vorzugsweise humanen Fibroblasten, da dann die extrazellulären Proteinteile z.T. natürlich glykosyliert und modifiziert werden.

Ein anderer Aspekt der Erfindung betrifft die Verwendung der intrazellulären - cytosolischen - Proteinsequenzen des CD30, die zur intrazellulären Signaltransduktion beitragen. Es ist naheliegend, daß diese Sequenzen für einen Abschnitt kodieren, welcher der Interaktion mit anderen Proteinen, die die weitere Signaltransduktion vermitteln, dient. Der an der intrazellulären Signaltransduktion beteiligte cytosolische Teil des CD30 wird, wie Vergleichsstudien, die Hydropathiekurve und *in-vitro*-Transkriptions/Translationsuntersuchungen zeigen, im wesentlichen kodiert von der Nucleotidsequenz (vgl. Figur 2) zwischen Position 1444 und 2007, also der Sequenz zwischen ¹⁴⁴⁴-CACCGGAGGG CCTGCA ... //... CTGCCTCTGGAAAG-₂₀₀₇. Bei diesem Aspekt der Erfindung steht gleichfalls die Synthese von rekombinanten CD30-Protein(abschnitt)en mit der cytosolischen Aktivität im Vordergrund und im weiteren die Entwicklung von Liganden und Bindungspartner, welche die cytosolische Aktivität von CD30 beeinflußen.

Die erfindungsgemäße Lehre beinhaltet ferner die Verwendung der obengenannten Nucleotidsequenzen zur Herstellung von transgenen Tieren, insbesondere Nagern, mit veränderter CD30-Antigen-Expression - mutiertem, insbesondere trunkiertem CD30, Über- oder Unterexpression an CD30, einer Expression von CD30 in anderen Zellen und Geweben als Lymphozyten, vollständigem Fehlen eines intakten Strukturgens für CD30, Konstitutiver oder induzierbarer Expression an CD30, einer Expression von CD30 an einer vorbestimmten Stelle bei der Differenzierung der Zellen. Derartige transgene Tiere sind hervorragende Modelle zur wissenschaftlichen Untersuchung von Lymphomen, zur Entwicklung von klinischen Test, zur Untersuchung von Blocker - und Induktor-Molekülen für den extrazellulären Rezeptorteil des CD30-Antigens.

Weitere bevorzugte Ausführungsformen der Erfindung sind in den Neben- und Unteransprüchen beschrieben.

Die durch die erfindungsgemäße Lehre zur Verfügung gestellten Mittel und Verfahren ermöglichen nunmehr den Nachweis von CD30-Antigen kodierenden DNAs und RNAs durch Hybridisierung bzw. die Lokalisierung des Gens auf dem/den Chromosom(en). Es kann somit untersucht werden, ob in den Zellen, in der Probe eine Transkription des CD30-Gens erfolgte und auch die Regulationsmechanismen zur Expression des CD30-Antigens.

Neben diesem mehr im wissenschaftlichen Bereich liegenden Nutzungsmöglichkeiten, erlauben insbesondere die neueren Hybridisierungsverfahren - die PCR-Reaktion (polymerase chain reaction), die in situ Hybridisierung an Gewebsschnitten und Chromosomen - eine weitere und sehr empfindliche Diagnose. Insbesondere ist diese Diagnostik nicht mehr anfällig gegenüber posttranslationalen Modifikationen und Prozessierungen.

Zumindest haben die Untersuchungen zur chromosomalen Lokalisierung des CD30-Strukturgens gezeigt, daß dieses auf der chromosomalen Bande 1p36 liegt. Das CD30-Gen liegt somit an einer fragilen Stelle im Chromosom (siehe: LeBeau (1986) Blood 67, 849-858), wo auch schon bei Morbus Hodgkin und non-Hodgkin-Lymphomen und anderen Neoplasien, wie Neuroblastomen und bösartigen Melanomen, chromosomale Abnormitäten, einschließlich von Virusinsertionen (EBV, HTLV I und II, HV6 und andere) festgestellt wurden. Hierbei ist besonders zu bemerken, daß bei non-Hodgkin-Lyphomen, Veränderung - Duplikationen, Translokationen, Deletionen, etc. - in diesem chromosomalen Bereich auftreten, die zusammen mit der Expression des CD30-Antigens mit einem besonders raschen Tumorwachstum einhergehen. Die erfindungsgemäß zur Verfügung gestellten Nucleotidsequenzen sind somit hervorragende diagnostische Mittel für die Frage, ob bei Patienten derartige chromosomale Veränderungen aufgetreten sind und mit dem Auftreten der Hodgkinschen Krankheit oder verwandter großzelliger anaplastischer Lymphome gerechnet werden muß.

Die erfindungsgemäß zur Verfügung gestellten Nucleotidsequenzen sind im weiteren auch für eine pränatale Diagnostik geeignet, insbesondere wenn in der Familie derartige Erkrankungen bereits aufgetretenen sind.

Weitere Vorteile, Ausführungsformen und Merkmale der Erfindung ergeben sich aus der nachfolgenden Beschreibung in Verbindung mit den Figuren und Darstellungen. Es zeigen:
Figur 1
   - A): klonierte cDNA-Abschnitte der CD30-Antigen-mRNA in Lambda gt11 ;
   - B): die für das CD30-Antigen kodierenden Bereiche (schattiertes Rechteck) auf der mRNA und die nicht-kodierenden Regionen (Linien) mit den Polyadenylierungsstellen (Sterne);
   - C): Restriktionskarte der cDNA mit verschiedenen Restriktionsenzymen;
   - D): Kartierung des CD30-Antigens nach den Epitopen der monoklonalen Antikörper Ki-1 und Ber-H2;
Figur 2 die Sequenz des nicht-transkripierten Stranges der DNA, des kodierenden Stranges der DNA, sowie wie die daraus abgeleitbare CD30-Proteinsequenz;
Figur 3 die Untersuchung verschiedener menschlicher Zellinien auf CD30-mRNAs;
Figur 4 ein Diagramm der hydrophoben und hydrophilen Eigenschaften der CD30-Aminosäuresequenz nach Kyte & Doolittle (1982) J. Mol. Biol., 157, 105-132;
Figur 5 den Sequenzvergleich von CD30 mit Sequenzen aus der NGF-Rezeptor-Proteinfamilie;
Figur 6 die Expression von CD30 in COS-Zellen.
   Immunpräzipitation von ¹²⁵J markierten Zellen:
   Laufspur 1: Markerproteine
   Laufspur 2: BER-H2 Präzipitat von CD30-5 transfizierten COS Zellen
   Laufspur 3: BER-H2 Präziptat von HUT 102 Zellen
   Laufspur 4: BER-H2 Präzipitat von mock (PCDM8) transfizierten COS Zellen

### Klonierung und Untersuchung der CD30-cDNA

Eine Lambda-gt11-cDNA-Genbank von HUT 102-Zellen (Poiesz et al. (1980) Proc. Natl. Acad. Sci. USA, 77, 7415-7419) bestehend aus ca. 200 000 Klonen wurde mit monoklonalen Antikörpern auf Hybridproteine mit CD30-Antigen getestet.

Die cDNA-Synthese nach Poly(A)⁺-RNA aus HUT 102-Zellen erfolgte gemäß Guebler & Hoffmann (1983) Gene 25, 263-269. Die cDNA wurde dazu abweichend mit einer Sepharose-CL4B-Säule größenselektiert (Eschenfeldt & Berger (1987) Methods in Enzymol., 152, 335-337). Das Immunoscreening erfolgte mit einem Gemisch der monoklonalen Antikörper Ki-1 und Ber-H2 gemäß Huynh et al. (1985) in "DNA Cloning", A Practical Approach, Bd. 1, 49-78 (Hrsg.: Glover D.M., IRL Press, Oxford, Washington DC.).

Ein Klon mit einer 909 Basenpaar langen cDNA produzierte ein Hybridpeptid, das der monoklonale Antikörper Ber-H2 erkannte.

Um einen Klon mit der gesamten Sequenz für CD30 zu erhalten, wurde eine pCDM8-Genbank aus HUT 102-Zellen hergestellt (Seed & Aruffo (1987) Proc. Natl. Acad. Sci. U.S.A., 84, 3365-3369; Aruffo & Seed (1987) Proc. Natl. Acad. Sci. U.S.A., 84, 8573-8577). Die cDNA wurde hierbei mit einem cDNA-Synthese-Kit der Firma Invitrogen synthesiert, dann mit BstXI-Linkern versehen und schließlich auf einem 1,5%igen (w/v) Nu-Sieve Gel der Firma FMC größenfraktioniert. cDNA mit mehr als 1 kBp wurde aus dem Gel isoliert und mit dem Vektor pCDM8 ligiert. Die Transformation erfolgt in E.coli-MC1061/p3 durch Elektroporation gemäß Dover et al (1988) Nucl. Acids. Res. 16, 6127 - 6145.

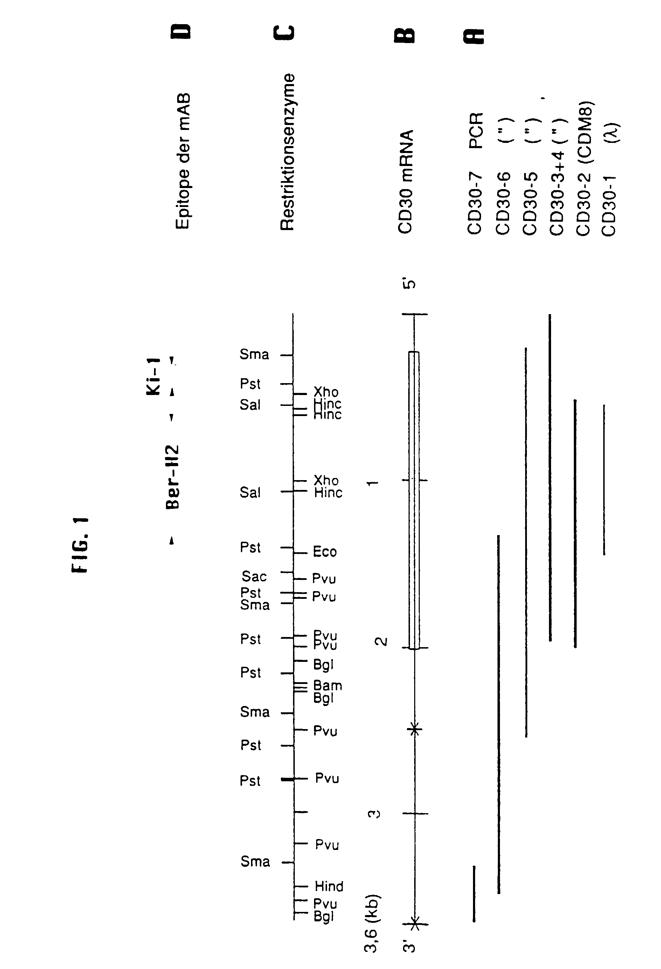

Die Koloniehybridisierungen erfolgten mit der durch das Immunoscreening der Lambda-gt11-Bank erhaltenen CD30-1-cDNA-Probe (Maniatis T., Fritsch E.F. Sambrook J. (1989) Molecular Cloning: A laboratory manual; Cold Spring Harbour Press, Cold Spring Harbour, New York). Dieser Ansatz lieferte 5 in den cDNA-Sequenzen überlappende Klone (Figur 1) mit 1,492 Bp (CD30-2), 1.905 Bp (CD30-3 und CD30-4), 2.342 Bp (CD30-5) und 2.242 (CD30-6). DNA-Sequenzierungen erfolgten mit dem Sequenase Sequencing Kit der Firma US Biochemicals mit synthetischen Oligonukleotidprimern (Sanger et al. (1977) Proc. Natl. Acad. Sci. U.S.A., 74, 5463-5467).

Um auch den 3'Poly(A)-Ende zu klonieren, wurde eine PCR-Reaktion gemäß dem RACE-Protokoll (rapid amplification of cDNA ends) an revers transkripierter Poly(A)-RNA aus HUT102-Zellen durchgeführt (Frohman et al. (1988) Proc. Natl. Acad. Sci. U.S.A., 87, 5459-5463). Die Amplifikation erfolgte in 35 Zyklen bestehend aus: 1 Min. Denaturieren, gefolgt von 45 Sek. Anlagerung der Primer bei 54°C und 1,5 Min. Elongieren bei 72°C, mit internen und eine Sall-Stelle aufweisenden Oligo(dT)-Primern. Dabei wurde der Klon CD30-7 mit einer 333 Basenpaar langen cDNA erhalten.

Die DNA des Klons CD30-5 im Vektor pGEM1 wurde bei der DSM - Deutsche Sammlung von Mikroorganismen und Zellkulturen - gemäß dem Budapester Vertrag unter der Nummer DSM 6833 hinterlegt.

Die Sequenz der CD30-cDNA und die daraus ableitbare Proteinsequenz sind in Figur 2 dargestellt. Das Signalpeptid und die transmembranen Domänen sind im Doppel unterstrichen, Polyadenylierungssignale sind einfach unterstrichen. Die Polyadenylierungstellen sind durch Pfeile markiert. Die homologen Untereinheiten in der extrazellulären Domäne sind gerahmt, potentielle N-Glykosylierungsstellen durch Sterne markiert, potentielle Phosphorylierungstellen durch folgende Abkürzungen hervorgehoben: TYR (Tyrosinkinase), PKC (Proteinkinase C), CK2 (Caseinkinase II), AMP (cAMP/cGMP-abhängige Kinase).

Die vollständige Nucleotidsequenz der CD30-CDNA weist 3.630 Basenpaare auf, wobei der G/C-Gehalt bei 62% liegt. Der offene Leserahmen geht von Nucleotid 231 bis Nucleotid 2015 - dem Terminationscodon. Das ATG-Codon zur Initiation der Translation ist von typischen Start-Sequenzen umgeben (Kozak (1986) Cell 44, 283-292). Der offene Leserahmen besteht aus zwei zu 77% homologen ca. 360 Bp langen Domänen (381-742 und 906-1270). Vor dem offenen Leserahmen befinden sich 230 Basenpaare nicht-translatierte Leader-Sequenz. Danach folgen 1.613 Basenpaare untranslatierte Sequenz mit einer kurzen palindromischen Sequenz zwischen Nucleotid 2867 und 2888. Die ungewöhnlich lange 5'-Leadersequenz ist für die Expression nicht notwendig, denn eine cDNA ohne diese Sequenz, z.B die aus Klon CD30-5 (siehe Beispiel 3), kann in COS-Zellen exprimiert werden.

Im untranslatierten 3'-Bereich befinden sich zwei Stellen für eine Polyadenylierung (durch einen Pfeil in Figur 2 markiert), denen die ungewöhnlichen poly(A)-Signalsequenzen TGTAAA und AATAAT vorstehen. (Birnstiel et al. (1985) Cell 41, 349-359); Bardwell et al. (1985) Cell 65, 125-133; Sheets et al (1990) Nucl. Acids. Res., 18, 5799-5805).

### Verwendung von CD30-Nucleotidsequenzen zur Untersuchung von Zellen, Geweben und Chromosomen

### BEISPIEL 1

Northernblot-Analysen: Die Isolierung der RNAs aus den Zellen und Geweben erfolgte nach dem Guanidiniumisothiocyanat-Cäsiumchlorid-Verfahren von Chirgwin et al (1979) Biochemistry, 18, 5294-5299. Die RNA wurde über eine Oligo(dT)-Cellulosesäule gemäß Swan et al (1972) Proc. Natl. Acad. Sci. USA, 69, 1408-1412 gereinigt. Die so erhaltene Poly(A)⁺-RNA wurde dann auf einem 1,2%igen (w/v) Agarosegel mit 2,2 Mol/L Formaldehyd gelelektrophoretisch aufgetrennt - 2 ug Poly(A)⁺-RNA pro Laufspur - auf Nitrocellulosemembranen übertragen und anschließend gegen ³²P-dCTP markierten CD30-DNA-lnserten hybridisiert. Die radioaktive Markierung der DNA erfolgte mit dem "DNA labeling kit" von Boehringer/Mannheim. Die Hybridisierungen erfolgte nach dem Verfahren von Thomas (1977) Proc. Natl. Acad. Sci. USA., 69, 1408-1412.

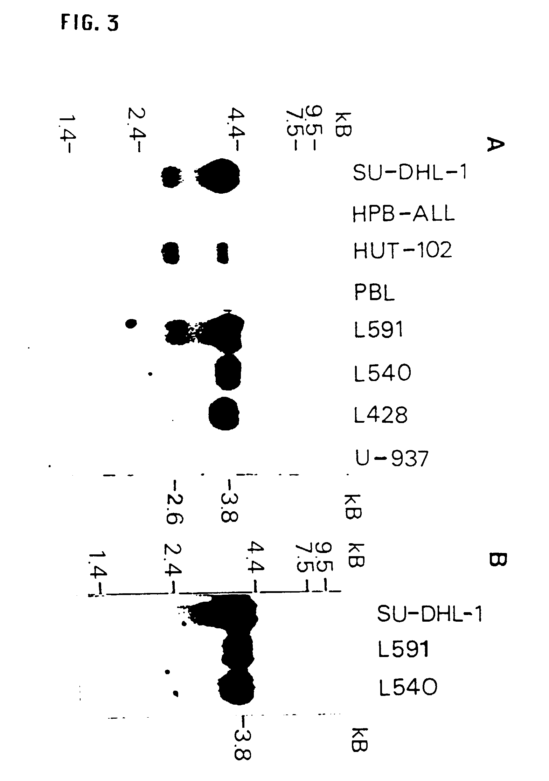

Eine derartige Northernblot-Analyse von RNA aus den menschlichen Zellinien HUT 102, SU-DHL-1 (Morgan et al (1989) Blood 73, 2155-2164), L-428 (Schaadt et al (1980) Int.J.Cancer 26, 723-731), L-591 (Diehl et al (1982) Cancer Treat. Rep. 66, 615-632), L-540 (Diehl et al (1981) J. Cancer Res. Clin. Oncol. 101, 111-124) Co (Jones et al. (1985) Hematol. Oncol. 3, 133-145), Ho (Kamesaki et al. (1986) Blood, 68, 285-292), U-937 (Sundström & Nilsson (1976) Int. J. Cancer 17, 565-577), MDA-MB-231 (Cailleau et al. (1974) J. Nat. Cancer Inst. 53, 661-674) und HPB-ALL (Boylston & Cosford (1985) Eur. J. Immunol. 15, 738-742) sowie - zur Kontrolle - von PBL-Zellen (periphären Blut-Lymphozyten)) ist in den Figuren 3A und B gezeigt; die Autoradiographie dauerte 4 Tage. Als Hybridisierungsprobe diente das CD30-5-Insert mit dem kodierenden Bereich (Fig 3A) und mit dem CD30-6/BgI1-Xba1-Fragment des nicht-translatierten 3'-Endes (3B).

In den untersuchten Zellinien können zwei RNAs nachgewiesen werden, eine größere mit ca. 3800 Nucleotiden und eine kleinere mit ca. 2600 Nucleotiden. Die RNAs beruhen wahrscheinlich auf verschiedenem Spleißen bei der Polyadenylierung.

Die Menge an CD30-kodierenden RNAs war am größten in der Zellinie SU-DHL-1 und in der aus einem Hodgkinschen Lymphom stammenden Zellinie L-591. Es folgten die aus Hodgkinschen Lymphomen stammenden Zellinien L-540, L-428, Ho, Co und schließlich die durch HTLV-1 transformierte Zellinie HUT 102. In Übereinstimmung mit dem Auftreten des CD30-Antigens waren keine RNA-Transkripte in periphären Blutlymphozyten sowie den Zellinien HPB-ALL, MDA-MB-231 und U-937 vorhanden. Dies zeigt die hohe Spezifität und Sensitivität der Hybridisierungsprobe.

### BEISPIEL 2

Chromosomen-Kartierung: Durch PHA stimulierte periphäre Blut-Lymphozyten-lsolate aus 2 gesunden Spenderpersonen wurden gemäß Stollmann et al. (1985), Br. J. Haematol., 60, 183-196, in Kultur durch Colchicin bei der Zellteilung in der Metaphase blockiert, die Chromosomen präpariert und daran eine in situ Hybridisierung gemäß Fonatsch et al. (1987), Recent Results Cancer Res., 117, 35-49, mit dem CD30-5/SmaI-cDNA-Fragment, das die Sequenz zwischen der Smal-Stelle an Position 224 und der Smal-Stelle an Position 1738 enthält, durchgeführt. Die DNA wurde durch Nick-Translation mit ³H-dCTP, ³H-dTTP radioaktiv markiert. Die spezifische Aktivität der Probe betrug 1,2 x 10⁷ dpm/ug DNA. 99 Metaphasen wurden ausgezählt.

Diese Untersuchungen ergaben, daß das CD30-Gen auf der Chromosomenbande 1p36 liegt. 21,6% von 162 Silberkörner lagen spezifisch in diesem Bereich. Der Chi-Quadratwerte sind hochsignifikant (P < 0,001).

### BEISPIEL 3

Expression von CD30-Antigen in COS-Zellen: Hierzu wurden COS-Zellen mit CD30-5-cDNA transfektiert und dann das CD30-Antigen aus diesen Zellen immunpräzipitiert. Als Expressionsvektor wurde der pCDM8-Vektor verwendet, in dem das CD30-cDNA-Stück einkloniert war. Die Transfektion erfolgte an COS-1-Zellen in Zellkultur mit dem DEAE-Dextran-Verfahren (Seed & Aruffo (1987) Proc.Natl.Acad.Sci.U.S.A., 84, 3365-3369). 48 Std. nach der Transfektion wurden die Zellen isoliert, mit Hilfe von IODO-Beads (Firma Pierce) radioaktiv markiert, solubilisiert, die betreffende Proteine immunpräziptiert und gelelektrophoretisch aufgetrennt.

Als Referenz wurden Oberflächenproteine von HUT 102-Zellen mit Hilfe von IODO-BEADS (hergestellt von der Firma Pierce) radioaktiv markiert und mittels Anti-Maus-lg beschichteten Agarosebeads isoliert (Schwarting et al. (1989) Blood 74, 1678-1689).

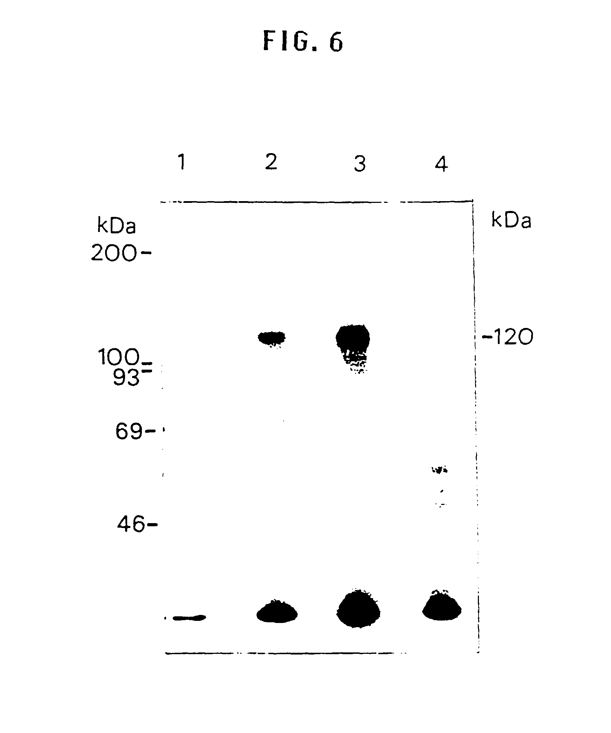

Figur 6 zeigt die elektrophoretische SDS-Gel-Analyse von mit monoklonalen Antikörper Ber-H2 immunpräzipitiertem CD30: Spur 1: Kontrolle; Spur 2: CD30-cDNA transfektierte COS-Zellen; Spur 3: aus ¹²⁵I-markierten HUT 102-Zellen; Spur 4: Kontrolle mit insertlosem Vektor transfizierte COS-Zellen. Stets wurde eine einzige Proteinbande mit einem relativen Molekulargewicht von 120 000 gefunden.

Ferner wurden immuncytologische APAAP (alkalische Phosphatase anti-alkalische Phosphatase) Färbungen (Cordell et al. (1984) J. Histochem. Cytochem., 32, 219-229) durchgeführt. Dadurch konnten die Epitope der monoklonalen Antikörper Ki-1 und Ber-H2 anhand des Vergleichs, welcher monoklonale Antikörper mit welchem Hybridprotein bzw. trunkiert exprimiertem Protein reagierte, und der aus der cDNA-Karte abgeleiteten Aminosäuresequenz kartiert werden. Durch Kombination der Daten aus lambda gt 11 Immunoscreening und APAAP Immunfärbung kann das Ki-1 Epitop in der extrazellulären Domäne (siehe Figur 1 D) zwischen dem Aminoterminus und der Aminosäure 93, das Ber-H2-Epitop zwischen den Aminosäuren 112 und 416 lokalisiert werden.

### Beispiel 4

Expression des CD30 Antigens in Kaninchenzellinien RK13 und R9ab für die Immunisierung von Kaninchen zur Herstellung polyklonaler Seren gegen CD30. Hierzu werden die Zellinien mit dem Vektor pCDM8-CD30-5 bzw. pRC/CMV-CD30-5 DEAE-Dextran transfiziert. Nach der Transfektion werden die Zellen geerntet und damit Kaninchen gemäß einem passenden Protokoll immunisiert.

### Beispiel 5

Expression des CD30 Antigens in Maus-Fibroblastenzellinien L929 und WOP zur Herstellung weiterer monoklonaler Antikörper mit Ratten und Mäusen gegen CD30. Hierzu werden die Maus-Fibroblasten mit dem Vektor pCDM8-CD30-5 bzw. pRC/CMV-CD30-5 durch die DEAE-Dextran Methode oder mittels Spheroblastenfusion (Sandri-Goldrin et al., Mol. Cell. Biol., 1, 743-752) transfiziert. Nach Transfektion werden mit den Zellen Mäuse bzw. Ratten nach einem passenden Protokoll immunisiert. Für die Herstellung der Hybridome werden die Milzen der Tiere präpariert und die so gewonnen Lymphozyten mit einer entsprechenden Maus-Myelom-Zellinie fusioniert (Köhler G, Milstein C, Nature (1975), 256, 495-497).

### Beispiel 6

Verwendung von rekombinant gewonnenem CD30-Antigen in RIAs, ELISAs, EIAs oder ähnlichen Immunodetektionssystemen zur Messung des bei bestimmten Erkrankungen in Seren vorkommenden, löslichen CD30-Antigens. Das CD30 kann hierfür durch Expression in Eukaryonten oder Prokaryonten unter Verwendung der angegebenen, möglicherweise leicht modifizierten Nucleinsäuresequenz, produziert werden. Nach affinitätschromatographischer Aufreinigung und Bestimmung des CD30 Gehaltes könnten solche Präparationen zur Standardisierung von CD30-Immuno-Assays dienen.

### Die CD30-Peptidsequenz und die sich daraus ergebenden Anwendungsmöglichkeiten der Erfindung:

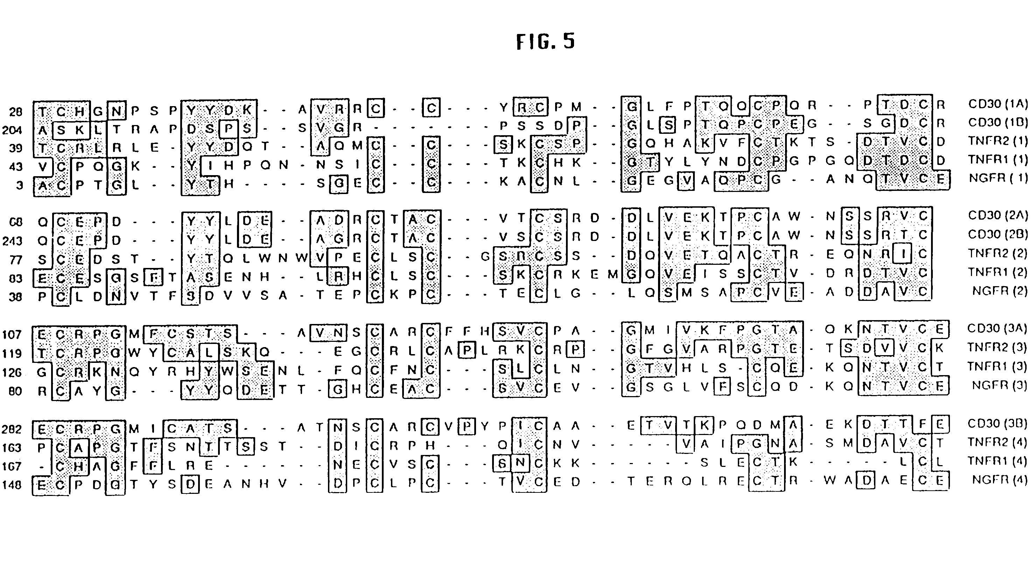

Der Vergleich der abgeleiteten Aminosäuresequenz für das CD30-Antigen zeigt, daß das CD30-Polypeptid anderen transmembranen Wachtumsfaktorrezeptoren gleicht. Insbesondere die erste - weniger die zweite - Cystein-reiche Untereinheit der extrazellulären Domäne gleicht den extrazellulären Domänen anderer bekannter Rezeptoren (siehe Figur 5). Gemeinsamkeiten bestehen im wesentlichen mit den menschlichen Nerve-Growth-Factor Rezeptor Proteinfamilie z.B. NGFR, TNFR-I, TNFR-II (Schall et al. (1990) Cell, 61, 361-370; Heller et al. (1990) Proc. Natl. Acad. Sci. U.S.A., 87, 6151-6155; Loetscher et al (1990) Cell, 61, 351-359; Kohno et al (1990) Proc. Natl. Acad. Sci. U.S.A., 87, 8331-8335; Engelmann et al. (1990) J. Biol. Chem., 265, 1531-1536; Smith et al (1990) Science 248, 1019-1023) und dem menschlichen Nervenwachstumsfaktor-Rezeptor (NGFR - nerve grwoth factor receptor) geringer Affinität (Johnson et al. (1986) Cell 47, 545-554).

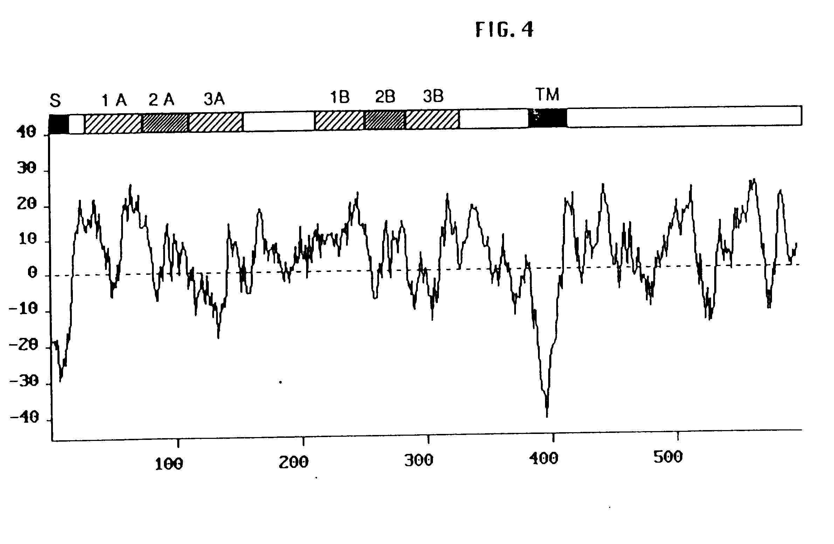

Die extrazelluläre Domäne von CD30 kann in 6 cysteinreiche Proteinmotive von 40 Aminosäuren unterteilt werden (siehe Figur 4), die - ausgenommen der verkürzten Motive 1B und 3B - jeweils 6 Cysteinreste aufweisen. Wie bei anderen Mitgliedern der NGFR Proteinfamilie sind die Positionen der Cysteine in diesen Unterdomänen hoch konserviert. Daneben bestehen noch Homologien zu den entsprechenden Bereichen folgender Rezeptoren: Humaninsulinrezeptor-ähnlicher-Rezeptor (engl. human insulin receptor-related receptor; Shier & Watt (1989) J. Biol. Chem., 264, 14605-14608)); transformierender-Wachstumsfaktor-β1 -Bindeprotein (engl.: transforming growth factor-β1 binding protein; Kanzaki et al. (1990) Cell, 61, 1051-1061); Maus-Interleukin-3-Rezeptor (Gorman et al. (1990) Proc. Natl. Acad. Sci. U.S.A., 87, 5459-5463); sevenless-Protein der Fruchtfliege (Michael et al. (1990) Proc. Natl. Acad. Sci. U.S.A., 87, 5351-5353).

Auf DNA-Ebene bestehen ferner Homologien zu anderen Wachstumsfaktor-Rezeptoren, insbesondere zu Human-Fibroblastenwachstumsfaktor-Rezeptor-4 (Partanen et al. (1991) EMBO J. 10, 1347-1354).

Die Resultate der Sequenzvergleiche ergeben somit, daß auch das CD30-Antigen höchstwahrscheinlich ein Rezeptor für einen Wachstumsfaktor ist. Deshalb liegt es nahe, die erfindungsgemäß zur Verfügung gestellten Nucleotid- und Proteinsequenzen von CD30 für Rezeptorstudien zu verwenden. Einerseits zur Untersuchung der Frage, welche Faktoren überhaupt an CD30 binden, in welcher Weise - kompetitiv oder konstitutiv - die Bindung beeinflußt werden kann und welche Auswirkungen die Bindung der Liganden für die Zelle hat.

Insbesondere bietet sich mit Hilfe der zur Verfügung gestellten Nucleotidsequenz die Schaffung transgener Tiere zur Untersuchung des Rezeptors und des damit verbundenen Signalsystems an.

## Patentansprüche

1. Nucleinsäure, insbesondere eine DNA oder RNA, enthaltend eine Nucleotidsequenz, die im wesentlichen der in Figur 2 gezeigten Sequenz für die cDNA des menschlichen lymphoiden Oberflächenantigens CD30 entspricht; DNA der Hinterlegung DSM 6833.

2. Nucleinsäure, insbesondere eine DNA oder RNA, enthaltend eine Nucleotidsequenz, die für ein Protein kodiert, dessen Aminosäuresequenz im wesentlichen der in Figur 2 gezeigten Sequenz des menschlichen lymphoiden Oberflächenantigens CD30 entspricht.

3. Nucleinsäure nach Anspruch 1 oder 2, enthaltend im wesentlichen eine Nucleotidsequenz, ausgewählt aus:
Nucleotidsequenz 277 bis 1359 beziehungsweise eine Nucleotidsequenz, die im wesentlichen die CD30-Proteinsequenz zwischen Aminosäure 19 und 379 kodiert;
Nucleotidsequenz 1444 bis 2007 beziehungsweise eine Nucleotidsequenz, die im wesentlichen die CD30-Proteinsequenz zwischen Aminosäure 408 und 595 kodiert;
Nucleotidsequenz, die im wesentlichen die CD30-Proteinsequenz zwischen Aminoterminus und Aminosäure 93 kodiert;
Nucleotidsequenz, die im wesentlichen die CD30-Proteinsequenz zwischen Aminosäure 112 und 416 kodiert.

4. Nucleinsäure nach Anspruch 1 oder 2, enthaltend im wesentlichen eine Nucleotidsequenz, ausgewählt aus:
der Nucleotidsequenz von Nucleotid 231 bis Nucleotid 2015 mit dem offenen Leserahmen der cDNA;
der Nucleotidsequenz von Nucleotid 224 bis Nucleotid 1738 mit dem SmaI-Fragment der cDNA;
der Nucleotidsequenz von Nucleotid 2468 bis Nucleotid 3622 mit dem BglI/XbaI-Fragment aus CDM8.

5. Klonierungsvektor, dadurch gekennzeichnet, daß er eine DNA gemäß einem der Ansprüche 1 bis 4 enthält.

6. Klonierungsvektor nach Anspruch 5, der ein oder mehrere Selektionsmarker aufweist.

7. Klonierungsvektor nach Anspruch 5 oder 6, wobei die DNA unter der Kontrolle eines konstitutiven und/oder induzierbaren Transkriptionspromotors steht.

8. Nucleinsäure nach einem der Ansprüche 1 bis 7, die ein oder mehrere laboranalytisch feststellbare Modifizierungen aufweist, ausgewählt aus radioaktive Atome, Farbstoffgruppen, eingeführte Epitope für auf Licht oder enzymatische Farbstoffreaktionen basierende Systeme zum Nachweis von Nucleinsäuren.

9. Nucleinsäure nach einem der Ansprüche 1 bis 8, die an einem makroskopischen Träger gebunden ist.

10. Zelle, die mit einer Nucleinsäure nach einem der Ansprüche 1 bis 7 transformiert ist.

11. Transformierte Zelle nach Anspruch 10, dadurch gekennzeichnet, daß sie das CD30-Antigen, ein Fragment des CD30-Antigens oder ein Hybridprotein mit dem CD30-Antigen oder Teilen davon exprimiert.

12. Transformierte Zelle nach Anspruch 10 oder 11, die eukaryotischen Ursprungs ist.

13. CD30-Antigen-Fragment, das kodiert wird von einer Nucleinsäure, die im wesentlichen der cDNA-Sequenz in Figur 2 zwischen Position 277 und Position 1359 beziehungsweise zwischen Position 1444 und Position 2007 entspricht oder ein rekombinantes Hybridprotein mit diesem CD30-Antigen-Fragment.

14. Rekombinantes Protein aus oder mit dem extrazellulären CD30-Antigenfragment nach Anspruch 13, das kodiert wird von einer Nucleotidsequenz, die im wesentlichen der cDNA-Sequenz in Figur 2 zwischen Position 277 und Position 1359 entspricht.

15. Rekombinantes Protein aus oder mit einem Teil des extrazellulären CD30-Antigenfragments nach Anspruch 13, wobei das Teil die CD30-Proteinsequenz zwischen Aminoterminus und Aminosäure 93 bzw. zwischen Aminosäure 112 und Aminosäure 416 mit dem Ki-1 bzw. Ber-H2 Epitop umfasst.

16. Rekombinantes Protein aus oder mit dem cytosolischen CD30-Antigenfragment nach Anspruch 13, das kodiert wird von einer Nucleotidsequenz, die im wesentlichen der cDNA-Sequenz in Figur 2 zwischen Position 1444 und Position 2007 entspricht.

17. Verfahren zur Herstellung von CD30-Antigen, einem Hybridprotein mit CD30-Antigen oder Fragmenten davon, oder von CD30-Antigenfragmenten, dadurch gekennzeichnet, daß eine Nucleotid- oder Aminosäuresequenz nach einem der Ansprüche 1 bis 4 verwendet wird.

18. Verfahren zur Herstellung von CD30-Antigen, einem Hybridprotein mit CD30-Antigen oder Fragment davon oder von CD30-Proteinfragmenten, dadurch gekennzeichnet, daß Zellen nach einem der Ansprüche 10 bis 12 in einem Nährmedium gezüchtet werden und dann die CD30-Moleküle isoliert werden.

19. Verwendung von CD30-Molekülen nach einem der Ansprüche 13 bis 16 als Immunogen zur Herstellung, Isolierung und Reinigung von Antikörpern und Antiseren.

20. Verwendung von CD30-Molekülen nach einem der Ansprüche 13 bis 16 zur Immunoabsorption.

21. Verwendung von CD30-Molekülen nach einem der Ansprüche 13 bis 16 und/oder dagegen gerichtete Antikörper als Faktoren in der Zellkultur.

22. Verwendung von CD30-Molekülen nach einem der Ansprüche 13 bis 16 zur Untersuchung und Isolierung von Molekülen mit der Eigenschaft, an CD30-Antigen zu binden.

23. Verwendung von Nucleinsäuren nach einem der Ansprüche 1 bis 7 zur Herstellung von transgenen Tieren, insbesondere transgenen Nagern, mit einer veränderten Expression an CD30-Antigen-ähnlichen Molekülen.

24. Verwendung von CD30-Molekülen nach einem der Ansprüche 13 bis 16 als Standard in einem ELISA, RIA oder in einem auf Antikörper basierenden Verfahren zum Nachweis von CD30-Antigen in Seren oder anderen Flüssigkeiten zu diagnostischen Zwecken.

25. Verfahren zur Durchführung eines ELISA, RIA oder eines anderen auf Antikörper basierenden Verfahrens zum Nachweis oder zur Bestimmung von CD30-Antigen, dadurch gekennzeichnet, daß man in einem ersten Schritt CD30-Moleküle mit einem Verfahren nach Anspruch 17 oder 18 gewinnt und eine bestimmte Menge davon als Standard oder Kontrollwert in dem Verfahren einsetzt.

26. Verfahren nach Anspruch 24, dadurch gekennzeichnet, daß es zur Diagnose von Morbus Hodgkin bzw. von anaplastischen großzelligen Lymphomen bzw. hystiocytischen Lymphomen oder einer eventuellen Aktivierung der Lymphozyten durch Viren eingesetzt wird.

## Claims

1. A nucleic acid, more particularly a DNA or RNA, containing a nucleotide sequence essentially corresponding to the sequence shown in Fig. 2 for a cDNA encoding human lymphoid CD30 antigen; DNA of the deposition DSM 6833.

2. A nucleic acid, more particularly a DNA or RNA, containing a nucleotide sequence encoding a protein which amino acid sequence essentially corresponds to the sequence shown in Fig. 2 for human lymphoid CD30 antigen.

3. A nucleic acid according to claim 1 or 2, essentially comprising a nucleotide sequence selected from
the nucleotide sequence from nucleotide 277 to 1359 or a nucleotide sequence essentially coding for the CD30 protein from amino acid 19 to 379;
the nucleotide sequence from nucleotide 1444 to 2007 or a nucleotide sequence essentially encoding the CD30 protein sequence from amino acid 408 to 595;
a nucleotide sequence essentially encoding the CD30 protein between the amino terminus and amino acid 93; and
a nucleotide sequence essentially encoding the CD30 protein between amino acid 112 and 416.

4. An isolated nucleic acid according to claim 1 or 2, essentially comprising a nucleotide sequence selected from
the nucleotide sequence between nucleotide 231 and nucleotide 2015 with the open reading frame of the cDNA;
the nucleotide sequence between nucleotide 224 and nucleotide 1738 with the SmaI fragment of the cDNA;
the nucleotide sequence from nucleotide 2468 to nucleotide 3622 with the BglI/XbaI-fragment of CDM8.

5. Cloning vector characterised in that it contains a DNA according to any one of claims 1 to 4.

6. A cloning vector according to claim 5 comprising one or more selection markers.

7. A cloning vector according to claim 5 or 6 wherein the DNA is under the control of a constitutive and/or an inducible transcription promoter.

8. A nucleic acid according to any of claims 1 to 7 and having one or more modifications detectable by laboratory analysis, selected from radioactive atoms, stain groups, or epitops introduced for systems based on light or enzymatic stain reactions for detection of nucleic acids.

9. A nucleic acid according to any of claims 1 to 8 bound to a macroscopic carrier.

10. A cell transformed with the nucleic acid according to any one of claims 1 to 7.

11. Transformed cell according to claim 10 characterised in that it expresses CD30-antigen or fragments thereof or a hybrid protein with CD30-antigen or fragments thereof.

12. Transformed cell according to claims 10 or 11 of eucaryotic origin.

13. CD30-antigen fragment encoded by a nucleic acid, essentially comprising the cDNA sequence of Fig. 2 between position 277 and position 1359 and position 1444 and position 2007, respectively, or a recombinant hybrid protein with this CD30-antigen fragment.

14. Recombinant protein of or with the extracellular CD30-antigen fragment of claim 13 which is encoded by a nucleotide sequence essentially comprising the cDNA sequence of Fig. 2 between position 277 and position 1359.

15. Recombinant protein of or containing a part of the extracellular CD30 antigen fragment according to claim 13 wherein said part comprises the CD30 protein sequence between the amino terminus and amino acid 93 or between amino acid 112 and amino acid 416 with the epitops of monoclonal antibodies Ki-1 and Ber-H2, respectively.

16. Recombinant protein of or with the cytosolic CD30-antigen fragment of claim 13 which is encoded by a nucleotide sequence essentially comprising the nucleotide sequence of the cDNA sequence of Fig. 2 between position 1444 and position 2007.

17. A method of producing CD30-antigen, a hybrid protein with CD30-antigen or fragments thereof, or a CD30-antigen fragment, characterised by using a nucleotide or amino acid sequence according to any of claims 1 to 4.

18. A method of producing CD30-antigen, a hybrid protein with CD30-antigen or fragments thereof or a CD30 protein fragment, characterised in that cells according to any of claims 10 to 12 are cultured in a nutrition medium and that CD30 molecules are isolated.

19. Use of CD30 molecules according to any of claims 13 to 15 as an immunogen for producing, isolating and purifying antibodies and antisera.

20. Use of CD30 molecules according to any of claims 13 to 16 for immuno-absorption.

21. Use of CD30 molecules according to any of claims 13 to 16 and/or antibodies thereof as factors in cell cultivation.

22. Use of CD30 molecules according to any of claims 13 to 16 for examination and isolation of molecules which are able to bind to CD30 antigen.

23. Use of nucleic acid according to any of claims 1 to 7 for producing transgenic animals, more particularly transgenic rodents with changed expression of CD30-antigen.

24. Use of CD30 molecules according to any of claims 13 to 16 for standardizing an ELISA, RIA or similar antibody-based process for detecting CD30-antigen in sera or other body fluids for diagnostic purposes.

25. Method for carrying out an ELISA, RIA or any other antibody-based process for detection or measurement of CD30-antigen, characterised in that in a first step CD30-molecules are obtained by a process according to claim 17 or 18 and in that a defined amount is used in that process as a standard or control value.

26. Method according to claim 25, characterised in that it is used for diagnosis of Morbus Hodgkin or anaplastic large cell lymphomas or histoid cystic lymphomas or an activation of lymphocytes by viruses.

## Revendications

1. Acide nucléique, en particulier un ADN ou un ARN, contenant une séquence nucléotidique qui correspond pour l'essentiel à la séquence représentée sur la figure 2 pour l'ADNc de l'antigène lymphoïde de surface CD30 humain ; ADN déposé sous la référence DSM 6833.

2. Acide nucléique, en particulier un ADN ou un ARN, contenant une séquence nucléotidique qui code pour une protéine dont la séquence d'amino-acides correspond pour l'essentiel à la séquence de l'antigène lymphoïde de surface CD30 humain, qui est représentée sur la figure 2.

3. Acide nucléique selon la revendication 1 ou 2, contenant essentiellement une séquence nucléotidique choisie parmi :
la séquence de nucléotides 277 à 1359 ou une séquence nucléotidique qui code essentiellement pour la séquence protéique CD30 entre les amino-acides 19 et 379 ;
la séquence de nucléotides 1444 à 2007 ou une séquence nucléotidique qui code essentiellement pour la séquence protéique CD30 entre les amino-acides 408 et 595 ;
une séquence nucléotidique qui code essentiellement pour la séquence protéique CD30 entre l'amino-terminus et l'amino-acide 93 ;
une séquence nucléotidique qui code essentiellement pour la séquence protéique CD30 entre les amino-acides 112 et 416.

4. Acide nucléique selon la revendication 1 ou 2, contenant essentiellement une séquence nucléotidique choisie parmi :
la séquence nucléotidique allant du nucléotide 231 au nucléotide 2015 avec le cadre ouvert de lecture de l'ADNc ;
la séquence nucléotidique allant du nucléotide 224 au nucléotide 1738 avec le fragment SmaI de l'ADNc ;
la séquence nucléotidique allant du nucléotide 2468 au nucléotide 3622 avec le fragment BglI/XbaI provenant de CDM8.

5. Vecteur de clonage caractérisé en ce qu'il contient un ADN selon l'une des revendications 1 à 4.

6. Vecteur de clonage selon la revendication 5, contenant un ou plusieurs marqueurs de sélection.

7. Vecteur de clonage selon la revendication 5 ou 6, l'ADN étant sous le contrôle d'un promoteur de transcription constitutif et/ou inductible.

8. Acide nucléique selon l'une des revendications 1 à 7, présentant une ou plusieurs modifications pouvant être constatées par des analyses en laboratoire, choisies parmi des atomes radioactifs, des groupes de colorants, des épitopes pour des systèmes basés sur la lumière ou des réactions enzymatiques avec des colorants pour déceler des acides nucléiques.

9. Acide nucléique selon l'une des revendications 1 à 8, qui est fixé sur un support macroscopique.

10. Cellule qui est transformée avec un acide nucléique selon l'une des revendications 1 à 7.

11. Cellule transformée selon la revendication 10, caractérisée en ce qu'elle exprime l'antigène CD30, un fragment de l'antigène CD30 ou une protéine hybride avec l'antigène CD30 ou des parties de celui-ci.

12. Cellule transformée selon la revendication 10 ou 11, qui est d'origine eucaryote.

13. Fragment d'antigène CD30 qui est codé par un acide nucléique qui correspond pour l'essentiel à la séquence d'ADNc de la figure 2, respectivement entre la position 277 et la position 1359 et entre la position 1444 et la position 2007, ou une protéine hybride recombinante avec ce fragment d'antigène CD30.

14. Protéine recombinante provenant du fragment d'antigène CD30 extracellulaire selon la revendication 13 ou avec celui-ci, qui est codée par une séquence nucléotidique qui correspond pour l'essentiel à la séquence d'ADNc de la figure 2 entre la position 277 et la position 1359.

15. Protéine recombinante provenant du fragment d'antigène CD30 extracellulaire selon la revendication 13 ou avec une partie de celui-ci, ladite partie comprenant la séquence protéique CD30 entre l'amino-terminus et l'amino-acide 93 ou entre l'amino-acide 112 et l'amino-acide 416 respectivement avec l'épitope Ki-1 ou Ber-H2.

16. Protéine recombinante provenant du fragment d'antigène CD30 cytosolique selon la revendication 13 ou avec celui-ci, qui est codée par une séquence nucléotidique qui correspond pour l'essentiel à la séquence d'ADNc de la figure 2 entre la position 1444 et la position 2007.

17. Procédé de préparation de l'antigène CD30, d'une protéine hybride avec l'antigène CD30 ou avec des fragments de celui-ci, ou de fragments d'antigène CD30, caractérisé en ce qu'on utilise une séquence de nucléotides ou d'amino-acides selon l'une des revendications 1 à 4.

18. Procédé de préparation de l'antigène CD30, d'une protéine hybride avec l'antigène CD30 ou avec un fragment de celui-ci, ou de fragments d'antigène CD30, caractérisé en ce qu'on cultive des cellules selon l'une des revendications 10 à 12 dans un milieu nutritif et ensuite, on isole les molécules de CD30.

19. Utilisation de molécules de CD30 selon l'une des revendications 13 à 16 comme immunogène pour la préparation, l'isolement et la purification d'anticorps et d'anti-sérums.

20. Utilisation de molécules de CD30 selon l'une des revendications 13 à 16 pour l'immuno-absorption.

21. Utilisation de molécules de CD30 selon l'une des revendications 13 à 16 et/ou d'anticorps dirigés contre celles-ci, comme facteurs pour la culture de cellules.

22. Utilisation de molécules de CD30 selon l'une des revendications 13 à 16 pour l'analyse et l'isolement de molécules ayant la propriété de se lier à l'antigène CD30.

23. Utilisation d'acides nucléiques selon l'une des revendications 1 à 7 pour la fabrication d'animaux transgéniques, en particulier de rongeurs transgéniques, avec une expression modifiée sur des molécules ressemblant à l'antigène CD30.

24. Utilisation de molécules de CD30 selon l'une des revendications 13 à 16 comme standard dans un test ELISA, un test RIA ou dans un procédé fondé sur des anticorps, pour la détection de l'antigène CD30 dans des sérums ou d'autres liquides à des fins de diagnostic.

25. Procédé de mise en oeuvre d'un test ELISA, RIA ou d'un autre procédé fondé sur des anticorps pour la détection ou la détermination de l'antigène CD30, caractérisé en ce que, lors d'une première étape, on obtient des molécules de CD30 au moyen d'un procédé selon la revendication 17 ou 18 et on en utilise une quantité définie comme standard ou comme valeur témoin dans le procédé.

26. Procédé selon la revendication 25, caractérisé en ce qu'on le met en oeuvre pour diagnostiquer la maladie de Hodgkin et des lymphomes anaplasiques à grandes cellules ou des lymphomes histiocytaires ou une éventuelle activation des lymphocytes par des virus.
